(19)

Europäisches
Patentamt
European
Patent Office
Office européen
des brevets

(11) **EP 3 643 322 A1**

(12) **EUROPEAN PATENT APPLICATION**

(43) Date of publication:
**29.04.2020 Bulletin 2020/18**

(51) Int Cl.:
*A61K 39/395* (2006.01)          *C07K 16/06* (2006.01)
*C07K 16/28* (2006.01)

(21) Application number: **18461619.1**

(22) Date of filing: **26.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Mabion SA**
**95-050 Konstantynow Lodzki (PL)**

(72) Inventors:
• **WIECZOREK, Maciej**
  **90-050 Konstantynow Lodzki (PL)**

• **JAROS, Slawomir**
  **90-050 Konstantynow Lodzki (PL)**
• **JAROS, Dorota**
  **90-050 Konstantynow Lodzki (PL)**
• **WASZAK, Lukasz**
  **90-050 Konstantynow Lodzki (PL)**

(74) Representative: **Zwicker, Jörk**
**ZSP Patentanwälte PartG mbB**
**Hansastraße 32**
**80686 München (DE)**

(54) **LOW AGGREGATE ANTI CD20 LIGAND FORMULATION**

(57)   The present invention relates to pharmaceutical compositions comprising a CD20 ligand, wherein the antibody in the composition has a low rate of aggregation. The invention further relates to the use of the pharmaceutical composition in the treatment of multiple sclerosis.

Figure 1

SEQUENCE LISTING

<110> Mabion S.A

<120> Low aggregate anti CD20 ligand formulation

<130> 131-4

<160> 6

<170> PatentIn version 3.5

<210> 1
<211> 11
<212> PRT
<213> Homo sapiens

<400> 1

Arg Ala Ser Ser Ser Val Ser Tyr Ile His Trp
1               5                   10


<210> 2
<211> 7
<212> PRT
<213> Homo sapiens

<400> 2

Thr Ser Asn Leu Ala Ser Gly
1               5


<210> 3
<211> 9
<212> PRT
<213> Homo sapiens

<400> 3

Gln Trp Thr Ser Asn Pro Pro Thr Phe
1               5


<210> 4
<211> 5
<212> PRT
<213> Homo sapiens

<400> 4

Ser Tyr Asn Met His
1               5


<210> 5
<211> 16
<212> PRT
<213> Homo sapiens

<400> 5

```
Ala Ile Tyr Pro Gly Asn Gly Asp Thr Ser Tyr Asn Gln Lys Phe Lys
1               5                   10                  15


<210>   6
<211>   8
<212>   PRT
<213>   Homo sapiens


<400>   6


Tyr Cys Ala Arg Ser Thr Tyr Tyr
1               5
```

**Description**

The present invention relates to pharmaceutical compositions comprising a CD20 ligand, wherein the antibody in the composition has a low rate of aggregation. The invention further relates to the use of the pharmaceutical composition in the treatment of multiple sclerosis.

**Background of the Invention**

Multiple sclerosis (MS) is a disease of the central nervous system and one of the most frequent causes of neurological disabilities. The disease has an autoimmunology background and is considered as T-cell mediated.

MS has different clinical phenotypes. The most frequent is the relapsing remitting (RRMS) form characterized by development of acute symptoms of relapses or relapses of neurological deficits followed by a complete or incomplete recovery. Rate of relapses and appearance of new MS lesions are the main factors to be taken under analysis for evaluation of efficacy of anti-inflamatory drugs used for MS treatment, but also there are other factors such as disability progression. After some time most patients develop a phenotype called secondary progressive multiple sclerosis (SPMS). It is characterized by progression of disability which is associated or not with superimposed relapses. The third type of MS is primary progressive multiple sclerosis (PPMS) in which there is slow progression of neurological disability and symptoms without relapses. The fourth type is called progressive-relapsing multiple sclerosis (PRMS). It is characterized by insidious disability progression from onset with some rare superimposed relapses (Castillo-Trivino et al., 2013, PLOS One 8(7), e66308).

B cells and humoral immunity are also considered as factors involved in MS progression. Such theory is supported by presence of antibodies and complement within active MS lesions, ectopic lymphoid follicles and B-cell related chemokines in the CNS and intrathecally produced immunoglobins. The use of CD20 ligands, such as the anti-CD20 antibody Rituximab, specifically targeting B-cells and depleting them, were therefore succesfully used in the treatment of relapsing MS (Naismith R.T. et al., 2010, Neurology 74, 1860-1867). In following studies the use of CD20 targeting antibodies as promising therapy for MS, in particular relapsing MS, was documented (Cross et al., 2012, TherAdvNeurolDisord 5[6], 311-319; Castillo-Trivino et al., 2013, PLOS One 8(7), e66308).

Therapeutic proteins such as antibodies are versatile drugs that can be tailored to suit countless therapeutic applications. A drawback of therapeutic proteins is however, that they may be recognized by the recipients immune system and provoke an immune response. These immune responses range from transient occurrences without much clinical significance to severe life-threatening conditions. In order to prevent severe side effects it is therefore warranted to reduce the immunogenicity of therapeutic proteins.

Proteins in solution have the ability to form aggregates with each other, which may have an impact on their biological activity. Effects of protein aggregation may not only lead to a decrease in activity of therapeutic proteins, but may also result in unwanted immune responses. In case of therapeutic protein formulations the formation of such protein aggregates can therefore influence the efficacy and safety profile of these drugs.

The effect of protein aggregation on the activity of a therapeutic protein was for example shown for interferon-beta. Runkel and colleagues compared the activity of two recombinant interferon-beta proteins that differed in their ability to form aggregates due to differences in their glycosylation pattern. They observed a significantly reduced activity for the recombinant interferon-beta protein that exhibited an increased aggregate formation as compared to the other interferon-beta protein, thereby demonstrating the effect of aggregate formation on the activity of a protein based drug (Runkel et al., 1998, PharmRes, 15(4):641-9).

In addition to effects on the activity of therapeutic proteins, aggregate formation may also affect the immunogenicity of these drugs. This can lead to decreased activity but also result in severe adverse effects of protein based drugs. A major safety concern regarding protein aggregates is their ability to increase the potential of proteins to elicit a severe immediate hypersensitivity response (anaphylaxis) or an immune response. While a role of protein aggregates in initiating an IgE response, which is the main pathomechanism underlying anaphylaxis, is not documented, it is well known that protein aggregates can modify an IgE response by degranulation of mast cells (Rosenberg, 2006, AAPS J. 8(3):E501-07), which promotes anaphylaxis.

In addition to immediate immune responses the increased immunogenicity of protein aggregates can result in the formation of anti-drug antibodies (ADA), such as drug-binding or drug-neutralizing antibodies. Treatment with human growth hormone (hGH) is a long known protein based drug therapy in which aggregate formation was shown to be related to an immune response to the therapeutic protein. Patients treated with hGH with a higher degree of aggregate formation did have a faster immune response after start of therapy by formation of antibodies binding hGH in direct comparison with patients receiving hGH with a lower amount of hGH aggregation. Furthermore, the immune response also differed between a persistent immune response in the patients receiving hGH with a higher level of aggregate formation as compared to only a transient immune response in patients receiving hGH with a lower level of aggregates. The presence of protein aggregates in protein based drug compositions can therefore have detrimental effects on the activity and increases the chances for severe adverse immunological reactions.

This importance of protein aggregates is also recognized by marketing authorization agencies such as the European Medicines Agency (EMA) as discussed in their *"Guideline on Immunogenicity assessment of therapeutic proteins"*, which specifically refers to the influence of protein aggregates on the development of an immune response on a therapeutic protein. Therapeutic protein compositions, in particular antibody-based drug compositions, with a reduced amount of protein aggregates are therefore needed to improve efficacy and safety of these drug compositions.

## Summary of the Invention

In a first aspect, the present invention provides a pharmaceutical composition comprising an antibody comprising in its light chain a LCDR1 with the amino acid sequence of SEQ ID NO: 1, a LCDR2 with the amino acid sequence of SEQ ID NO: 2, and a LCDR3 with the amino acid sequence of SEQ ID NO: 3 and comprising in its heavy chain a HCDR1 with the amino acid sequence of SEQ ID NO: 4, a HCDR2 with the amino acid sequence of SEQ ID NO: 5, and a HCDR3 with the amino acid sequence of SEQ ID NO: 6, wherein less than 0.7 wt% of the antibody in the composition is aggregated, and/or wherein the antibody is obtainable by a process comprising:

(i) incubating a mammalian cell comprising a nucleic acid encoding the antibody in a culture medium in suspension culture, wherein the mammalian cell secretes the antibody into the culture medium;
(ii) separating the antibody from the culture medium;
(iii) solubilizing the antibody in a buffer; and
(iv) purifying the antibody by binding to a cation-exchange chromatography resin and eluting a fraction of the antibody from the resin, comprising less than 10% of basic charge variants of the antibody;

wherein
the pH of the buffer used in solubilizing and purifying the antibody is between 5 and 8, the conductivity is between 1 and 50 mS/cm.

In a second aspect, the present invention provides the pharmaceutical composition of the first aspect of the invention for use in the treatment of multiple sclerosis.

## List of Figures

In the following, the content of the figures comprised in this specification is described. In this context please also refer to the detailed description of the invention above and/or below.

**Figure 1: Process Flow chart.** WCB (Working Cell Bank), ICC (Inoculum Cell Culture), BCC (Bioreactor Cell Culture), CH (Cell Harvest), PAAC (Protein A Affinity Chromatography), LPVI (Low pH Virus Inactivation), CEX (Cation Exchange Chromatography), AEX 1 (Anion Exchange Chromatography 1), UF (Ultrafiltration), AEX 2 (Anion Exchange Chromatography 2), VF (Virus Filtration), UFDF (Ultrafiltration with Diafiltration), AP (Adding polysorbate and dilution), SF (Sterile Filtration), FF (Final filling), LP (Packaging and Labelling)

**Figure 2: Purification method.** Flow chart presenting method of purification of the product related impurities.

**Figure 3: Aggregate content of MabThera and Mabion CD20DP (SEC-HPLC).** The aggregates level in MabionCD20DP and MabThera batches by SEC-HPLC. Aggregate content for MabionCD20DP batches analyzed is below 0.60%.

**Figure 4: HMW species.** The HMW species level in MabionCD20DP and MabThera batches by SEC-MALLS.

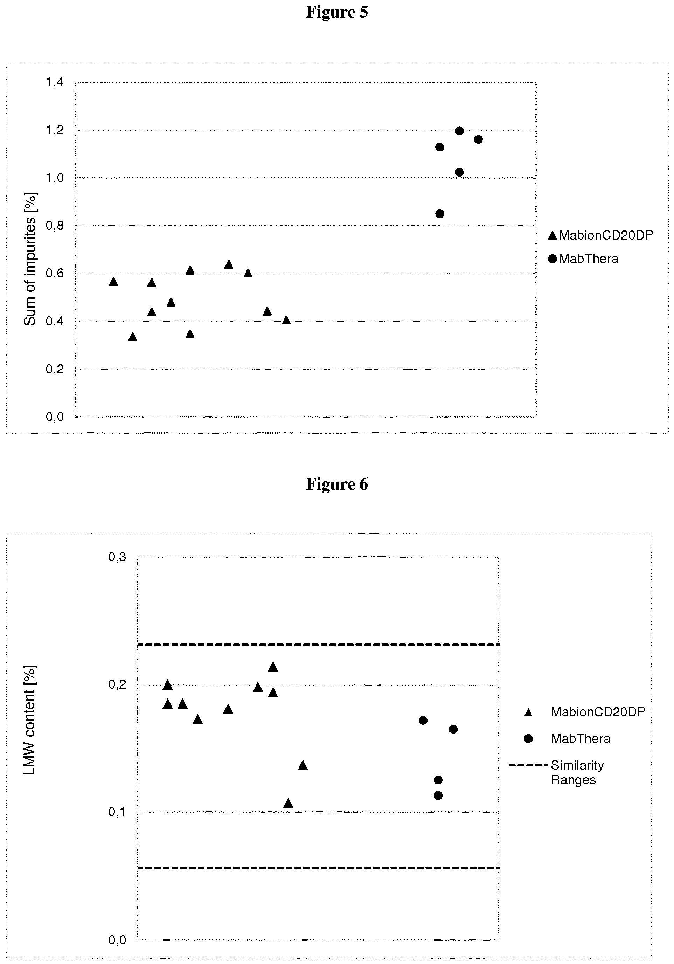

**Figure 5: Impurities.** The sum of aggregates level (sum of HMW species (Fig.4) and LMW süecies (Fi. 6)) in MabionCD20DP and MabThera batches by SEC-MALLS.

**Figure 6: LMW species.** The LMW species level in MabionCD20DP and MabThera batches by SEC-MALLS (similarity ranges based on results obtained for 5 MabThera batches ± 3 SD)

## Detailed Descriptions of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms:

(IUPAC Recommendations)", Leuenberger, H.G.W, Nagel, B. and Klbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages, different aspects of the invention are defined in more detail. Each aspect so defined may be combined with any other aspect or aspects unless clearly indicated to the contrary. In particular, any feature indicated as being optional, preferred or advantageous may be combined with any other feature or features indicated as being optional, preferred or advantageous.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Definitions

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

The term "about" when used in connection with a numerical value is meant to encompass numerical values within a range having a lower limit that is 5% smaller than the indicated numerical value and having an upper limit that is 5% larger than the indicated numerical value.

As used herein, "CD20" is an activated-glycosylated phosphoprotein expressed on the surface of all B-cells. The sequence of human CD20 is indicted in GenBank reference NP_690605.

The term "ligand" as used herein includes but is not limited to antigen binding protein antibodies and fragments thereof.

The term "antigen binding protein" as used herein, refers to immunoglobulin molecules and immunologically active portions of immunoglobulin molecules, i.e. molecules that contain an antigen-binding site that immunospecifically binds an antigen. Also comprised are immunoglobulin-like proteins that are selected through techniques including, for example, phage display to specifically bind to a target molecule or target epitope. In assessing the binding and/or specificity of an antigen binding protein, e.g., an antibody or immunologically functional fragment thereof, an antibody or fragment can substantially inhibit binding of a ligand to its binding partner when an excess of antibody reduces the quantity of binding partner bound to the ligand by at least about 1-20%, 20-30%, 30-40%, 40-50%, 50-60%, 60-70%, 70-80%, 80-85%, 85-90%, 90-95%, 95-97%, 97-98%, 98-99% or more (e.g. as measured in an in vitro competitive binding assay). The neutralizing ability may be described in terms of an $IC_{50}$ or $EC_{50}$ value.

The "$IC_{50}$" value refers to the half maximal inhibitory concentration of a substance and is thus a measure of the effectiveness of a substance in inhibiting a specific biological or biochemical function. The values are typically expressed as molar concentration. The $IC_{50}$ of a drug can be determined in functional antagonistic assays by constructing a dose-response curve and examining the inhibitory effect of the examined substance at different concentrations. Alternatively, competition binding assays may be performed in order to determine the $IC_{50}$ value. Typically, inhibitory antibodies of the present invention exhibit an $IC_{50}$ value of between 50 nM - 1 pM, more preferably 10 nM to 10 pM, and even more preferably between 1 nM and 50 pM, i.e. 50 nM, 10 nM, 1 nM, 900 pM, 800 pM, 700 pM, 600 pM, 500 pM, 400 pM, 300 pM, 200 pM, 100 pM, 50 pM, or 1 pM.

The "$EC_{50}$" value refers to half maximal effective concentration of a substance and is thus a measure of the concentration of said substance which induces a response halfway between the baseline and maximum after a specified exposure time. It is commonly used as a measure of drug's potency. The $EC_{50}$ of a graded dose response curve therefore represents the concentration of a substance where 50% of its maximal effect is observed. The $EC_{50}$ of a quantal dose response curve represents the concentration of a compound where 50% of the population exhibit a response, after a specified exposure duration. Typically, inhibitory antibodies of the present invention exhibit an $EC_{50}$ value of between 50 nM to 1 pM, more preferably 10 nM to 10 pM, and even more preferably between 1 nM and 50 pM, i.e. 50 nM, 10 nM, 1 nM, 900 pM, 800 pM, 700 pM, 600 pM, 500 pM, 400 pM, 300 pM, 200 pM, 100 pM, 50 pM, or 1 pM.

The term "antibody" typically refers to a glycoprotein comprising at least two heavy (H) chains and two light (L) chains inter-connected by disulfide bonds, or an antigen-binding portion thereof. The term "antibody" also includes all recombinant forms of antibodies, in particular of the antibodies described herein, e.g. antibodies expressed in prokaryotes, unglycosylated antibodies, and any antigen-binding antibody fragments and derivatives as described below. Each heavy chain is comprised of a heavy chain variable region (abbreviated herein as VH or $V_H$) and a heavy chain constant region. Each light chain is comprised of a light chain variable region (abbreviated herein as VL or $V_L$) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The term "antigen-binding fragment" of an antibody (or simply "fragment"), as used herein, refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding fragment" of an antibody include (i) Fab fragments, monovalent fragments consisting of the VL, VH, CL and CH domains; (ii) F(ab')$_2$ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is a binding-domain immunoglobulin fusion protein comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies. Further examples of "antigen-binding fragments" are so-called microantibodies, which are derived from single CDRs. For example, Heap et al., 2005, describe a 17 amino acid residue microantibody derived from the heavy chain CDR3 of an antibody directed against the gp120 envelope glycoprotein of HIV-1. Other examples include small antibody mimetics comprising two or more CDR regions that are fused to each other, preferably by cognate framework regions. Such a small antibody mimetic comprising $V_H$ CDR1 and $V_L$ CDR3 linked by the cognate $V_H$ FR2 has been described by Qiu et al., 2007.

Thus, the term "antibody or antigen-binding fragment thereof", as used herein, refers to immunoglobulin molecules and immunologically active portions of immunoglobulin molecules, i.e. molecules that contain an antigen-binding site that immunospecifically binds an antigen. Also comprised are immunoglobulin-like proteins that are selected through techniques including, for example, phage display to specifically bind to a target molecule or target epitope, e.g. to CD20. The immunoglobulin molecules of the invention can be of any type (e.g., IgG, IgE, IgM, IgD, IgA and IgY), class (e.g., IgG1, IgG2, preferably IgG2a and IgG2b, IgG3, IgG4, IgA1 and IgA2) or subclass of immunoglobulin molecule.

Antibodies and antigen-binding fragments thereof usable in the invention may be from any animal origin including birds and mammals. Preferably, the antibodies or fragments are from human, chimpanzee, rodent (e.g. mouse, rat, guinea pig, or rabbit), chicken, turkey, pig, sheep, goat, camel, cow, horse, donkey, cat, or dog origin. It is particularly preferred that the antibodies are of human or murine origin. Antibodies of the invention also include chimeric molecules in which an antibody constant region derived from one species, preferably human, is combined with the antigen binding site derived from another species, e.g. mouse. Moreover antibodies of the invention include humanized molecules in which the antigen binding sites of an antibody derived from a non-human species (e.g. from mouse) are combined with constant and framework regions of human origin.

As used herein, "human antibodies" include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo). Human antibodies of the invention include antibodies isolated from human immunoglobulin libraries or from animals transgenic for one or more human immunoglobulin and that do not express endogenous immunoglobulins, as described for example in U.S. Patent No. 5,939,598 by Kucherlapati & Jakobovits.

The term "monoclonal antibody" as used herein refers to a preparation of antibody molecules of single molecular composition. A monoclonal antibody displays a single binding specificity and affinity for a particular epitope. In one embodiment, the monoclonal antibodies are produced by a hybridoma which includes a B cell obtained from a non-human animal, e.g. mouse, fused to an immortalized cell.

The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g. from a transfectoma, (c) antibodies isolated from a recombinant, combinatorial antibody library, and (d) antibodies prepared, expressed, created or isolated by any other means that involve splicing of immunoglobulin gene sequences to other DNA sequences.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another species or class. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human is less likely to elicit an immune response from a human subject when the antibodies are injected than would the constant region from a non-human source. However, the definition is not limited to this particular example.

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen-binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

Different methods for humanizing antibodies are known to the skilled person, as reviewed by Almagro & Fransson, 2008, the content of which is herein incorporated by reference in its entirety. The review article by Almagro & Fransson is briefly summarized in the following. Almagro & Fransson distinguish between rational approaches and empirical approaches. Rational approaches are characterized by generating few variants of the engineered antibody and assessing their binding or any other property of interest. If the designed variants do not produce the expected results, a new cycle of design and binding assessment is initiated. Rational approaches include CDR grafting, Resurfacing, Superhumanization, and Human String Content Optimization. In contrast, empirical approaches are based on the generation of large libraries of humanized variants and selection of the best clones using enrichment technologies or high-throughput screening. Accordingly, empirical approaches are dependent on a reliable selection and/or screening system that is able to search through a vast space of antibody variants. In vitro display technologies, such as phage display and ribosome display fulfill these requirements and are well-known to the skilled person. Empirical approaches include FR libraries, Guided selection, Framework-shuffling, and Humaneering.

A "bivalent antibody" comprises two antigen binding sites. Bivalent antibodies may be monospecific or bispecific. In case, the bivalent antibody is monospecific, the two binding sites of the antibody have the same antigen specificities. A "bispecific" or "bifunctional" antigen binding protein or antibody is a hybrid antigen binding protein or antibody, respectively, having two different antigen binding sites. The two binding sites of a bispecific antigen binding protein or antibody bind to two different epitopes residing either on the same or on different antigens. Bispecific antigen binding proteins and antibodies are a species of multispecific antigen binding protein antibody and can be produced by a variety of methods including, but not limited to, fusion of hybridomas, chemical linking of IgG or IgG fragments such as Fab', or by genetic means. See, e.g., Songsivilai and Lachmann, 1990, Clin. Exp. Immunol. 79:315-321; Kostelny et al., 1992, J. Immunol. 148:1547-1553; Kontermann, 2014, MAbs 4:182-197.

A "trifunctional antibody" is a type of bispecific antibody which comprises the two binding sites targeting different antigens as well as an intact Fc-part which can bind to an Fc receptor on accessory cells (e.g. monocytes/macrophages, natural killer cells, dendritic cells or other). For example, a trifunctional antibody may comprise a binding site targeting an epitope on the surface of a cancer cell, the second binding site may target an epitope on the surface of a T cell (e.g. CD3) and the Fc-part may bind to the Fc receptor on the surface of a macrophage. Such trifunctional antibody is thus able to link T cells and macrophages to the tumor cells, leading to their destruction.

Papain digestion of antibodies produces two identical antigen binding fragments, called "Fab fragments" (also referred to as "Fab portion" or "Fab region") each with a single antigen binding site, and a "Fc fragment" (also referred to as "Fc portion" or "Fc region") whose name reflects its ability to crystallize readily. The crystal structure of the human IgG Fc region has been determined (Deisenhofer (1981) Biochemistry 20:2361-2370). In IgG, IgA and IgD isotypes, the Fc region is composed of two identical protein fragments, derived from the CH2 and CH3 domains of the antibody's two heavy chains; in IgM and IgE isotypes, the Fc regions contain three heavy chain constant domains (CH2-4) in each polypeptide chain. In addition, smaller immunoglobulin molecules exist naturally or have been constructed artificially. The term "Fab' fragment" refers to a Fab fragment additionally comprise the hinge region of an Ig molecule whilst "F(ab')2 fragments" are understood to comprise two Fab' fragments being either chemically linked or connected via a disulfide bond. Whilst "single domain antibodies (sdAb)" (Desmyter et al. (1996) Nat. Structure Biol. 3:803-811) and "Nanobodies" only comprise a single VH domain, "single chain Fv (scFv)" fragments comprise the heavy chain variable domain joined via a short linker peptide to the light chain variable domain (Huston et al. (1988) Proc. Natl. Acad. Sci. USA 85, 5879-5883). Divalent single-chain variable fragments (di-scFvs) can be engineered by linking two scFvs (scFvA-scFvB). This can be done by producing a single peptide chain with two $V_H$ and two $V_L$ regions, yielding "tandem scFvs" ($V_H$A-$V_L$A-$V_H$B-$V_L$B). Another possibility is the creation of scFvs with linkers that are too short for the two variable regions to fold together, forcing scFvs to dimerize. Usually linkers with a length of 5 residues are used to generate these dimers. This type is known as "diabodies". Still shorter linkers (one or two amino acids) between a $V_H$ and $V_L$ domain lead to the formation of monospecific trimers, so-called "triabodies" or "tribodies". Bispecific diabodies are formed by expressing to chains with the arrangement $V_H$A-$V_L$B and $V_H$B-$V_L$A or $V_L$A-$V_H$B and $V_L$B-$V_H$A, respectively. Single-chain diabodies (scDb) comprise a $V_H$A-$V_L$B and a $V_H$B-$V_L$A fragment which are linked by a linker peptide (P) of 12-20 amino acids, preferably 14 amino acids, ($V_H$A-$V_L$B-P-$V_H$B-$V_L$A). "Bi-specific T-cell engagers (BiTEs)" are fusion proteins consisting of two scFvs of different antibodies wherein one of the scFvs binds to T cells via the CD3 receptor, and the other to a tumor cell via a tumor specific molecule (Kufer et al. (2004) Trends Biotechnol. 22:238-244). Dual affinity retargeting molecules ("DART" molecules) are diabodies additionally stabilized through a C-terminal disulfide bridge.

As used herein, the term "antibody-like protein" or immunoglobulin-like protein refers to a protein that has been engineered (e.g. by mutagenesis of loops) to specifically bind to a target molecule. Typically, such an antibody-like protein comprises at least one variable peptide loop attached at both ends to a protein scaffold. This double structural constraint greatly increases the binding affinity of the antibody-like protein to levels comparable to that of an antibody. The length of the variable peptide loop typically consists of 10 to 20 amino acids. The scaffold protein may be any protein having good solubility properties. Preferably, the scaffold protein is a small globular protein. Antibody-like proteins include without limitation affibodies, anticalins, and designed ankyrin repeat proteins (for review see: Binz H.K. et al. (2005) Engineering novel binding proteins from nonimmunoglobulin domains. Nat. Biotechnol. 23(10): 1257-1268). Antibody-like proteins can be derived from large libraries of mutants, e.g. be panned from large phage display libraries and can be isolated in analogy to regular antibodies. Also, antibody-like binding proteins can be obtained by combinatorial mutagenesis of surface-exposed residues in globular proteins. Antibody-like proteins are sometimes referred to as "peptide aptamers".

Thus, "antibodies and antigen-binding fragments thereof' suitable for use in the present invention include, but are not limited to, polyclonal, monoclonal, monovalent, bispecific, heteroconjugate, multispecific, recombinant, heterologous, heterohybrid, chimeric, humanized (in particular CDR-grafted), deimmunized, or human antibodies, Fab fragments, Fab' fragments, F(ab')₂ fragments, fragments produced by a Fab expression library, Fd, Fv, disulfide-linked Fvs (dsFv), single chain antibodies (e.g. scFv), diabodies or tetrabodies (Holliger P. et al. (1993) Proc. Natl. Acad. Sci. U.S.A. 90(14), 6444-6448), nanobodies (also known as single domain antibodies), anti-idiotypic (anti-Id) antibodies (including, e.g., anti-Id antibodies to antibodies of the invention), and epitope-binding fragments of any of the above. The antibodies described herein are preferably isolated. An "isolated antibody" as used herein, is intended to refer to an antibody which is substantially free of other antibodies having different antigenic specificities (e.g., an isolated antibody that specifically binds to CD20 is substantially free of antibodies that specifically bind antigens other than CD20). An isolated antibody that specifically binds to an epitope, isoform or variant of human CD20 may, however, have cross-reactivity to other related antigens, e.g. from other species (e.g. CD20 species homologs, such as rat CD20). Moreover, an isolated antibody may be substantially free of other cellular material and/or chemicals. In one embodiment of the invention, a combination of "isolated" monoclonal antibodies relates to antibodies having different specificities and being combined in a well-defined composition.

The term "active agent" as used herein, refers to any therapeutic activity an agent may exhibit.

The term "compete" when used in the context of antigen binding proteins that compete for the same epitope means competition between antigen binding proteins as determined by an assay in which the antigen binding protein (e.g., antibody or immunologically functional fragment thereof) being tested prevents or inhibits (e.g., reduces) specific binding of a reference antigen binding protein (e.g., a ligand, or a reference antibody) to a common antigen (e.g., CD20 or a fragment thereof). Numerous types of competitive binding assays can be used to determine if one antigen binding protein competes with another, for example: solid phase direct or indirect radioimmunoassay (RIA), solid phase direct or indirect enzyme immunoassay (EIA), sandwich competition assay (see, e.g., Stahli et al., 1983, Methods in Enzymology 9:242-253); solid phase direct biotin-avidin EIA (see, e.g., Kirkland et al., 1986, J. Immunol. 137:3614-3619) solid phase direct labeled assay, solid phase direct labeled sandwich assay (see, e.g., Harlow and Lane, 1988, Antibodies, A Laboratory Manual, Cold Spring Harbor Press); solid phase direct label RIA using 1-125 label (see, e.g., Morel et al., 1988, Molec. Immunol. 25:7-15); solid phase direct biotin-avidin EIA (see, e.g., Cheung, et al., 1990, Virology 176:546-552); and direct labeled RIA (Moldenhauer et al., 1990, Scand. J. Immunol. 32:77-82). Typically, such an assay involves the use of purified antigen bound to a solid surface or cells bearing either of these, an unlabeled test antigen binding protein and a labeled reference antigen binding protein. Competitive inhibition is measured by determining the amount of label bound to the solid surface or cells in the presence of the test antigen binding protein. Usually the test antigen binding protein is present in excess. Antigen binding proteins identified by competition assay (competing antigen binding proteins) include antigen binding proteins binding to the same epitope as the reference antigen binding proteins and antigen binding proteins binding to an adjacent epitope sufficiently proximal to the epitope bound by the reference antigen binding protein for steric hindrance to occur. Usually, when a competing antigen binding protein is present in excess, it will inhibit (e.g., reduce) specific binding of a reference antigen binding protein to CD20 or an extracellular fragment thereof by at least about 40-45%, 45-50%, 50-55%, 55-60%, 60-65%, 65-70% or 70-75%, such as about 75% or more. In some instances, binding is inhibited by at least about 80-85%, 85-90%, 90-95% or 95-97%, such as about 97% or more.

The term "binding" according to the invention preferably relates to a specific binding. The term "binding affinity" generally refers to the strength of the sum total of noncovalent interactions between a single binding site of a molecule (e.g., an antibody) and its binding partner (e.g., target or antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant ($K_d$). "Specific binding" means that a binding moiety (e.g. an antibody) binds stronger to a target such as an epitope for which it is specific compared to the binding to another target. A binding moiety binds stronger to a first target compared to a second target if it binds to the first target with a dissociation constant ($K_d$) which is lower than the dissociation constant for the second target. The dissociation constant ($K_d$) for the target to which the binding moiety binds specifically is more than 10-fold, preferably more than 20-fold, more preferably more than 50-fold, even more preferably more than 100-fold, 200-fold, 500-fold or 1000-fold lower than the dissociation constant ($K_d$) for the target to which the binding moiety does not bind specifically.

Accordingly, the term "Kd" (measured in "mol/L", sometimes abbreviated as "M") is intended to refer to the dissociation equilibrium constant of the particular interaction between a binding moiety (e.g. an antibody or fragment thereof) and a target molecule (e.g. an antigen or epitope thereof). Affinity can be measured by common methods known in the art, including but not limited to surface plasmon resonance based assay (such as the BIAcore assay); quartz crystal microbalance assays (such as Attana assay); enzyme-linked immunoabsorbent assay (ELISA); and competition assays (e.g. RIA's). Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

Typically, ligands according to the invention bind with a sufficient binding affinity to their target, for example, with a Kd value of between 500 nM-1 pM, i.e. 500 nM, 450 nM, 400nM, 350 nM, 300nM, 250 nM, 200nM, 150 nM, 100nM, 50 nM, 10 nM, 1 nM, 900 pM, 800 pM, 700 pM, 600 pM, 500 pM, 400 pM, 300 pM, 200 pM, 100 pM, 50 pM, or 1pM, if measured by surface plasmon resonance based assay (such as the BIAcore assay.

The term "basic charge variants of the antibody" refers to antibody variants that have an isoelectric point (pI) higher than the pI of the main variants of an antibody. Any method that can differentiate protein charge can be used to determine the charge variants of an antibody. Non-limiting examples of methods that can be used are cation exchange chromatography and size exclusion chromatography. The main peak in a diagram of protein charges represents the main variants of an antibody, whereas variants with a higher pI are referred to as basic charge variants.

"Pharmaceutically acceptable" means approved by a regulatory agency of the Federal or a state government or listed in the U.S. Pharmacopeia, European Pharmacopeia (Ph. Eur.) or other generally recognized pharmacopeia for use in animals, and more particularly in humans.

The term "carrier", as used herein, refers to a diluent, adjuvant, excipient, or vehicle with which the therapeutic agent is administered. Such pharmaceutical carriers can be sterile liquids, such as saline solutions in water and oils, including those of petroleum, animal, vegetable or synthetic origin, such as peanut oil, soybean oil, mineral oil, sesame oil and the like. A saline solution is a preferred carrier when the pharmaceutical composition is administered intravenously.

Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. The compounds of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the patient. The formulation should suit the mode of administration.

Embodiments

In the following different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In the work leading to the present invention, it was surprisingly shown that therapeutic antibodies in pharmaceutical compositions can be produced with a very low level of aggregation without any additional steps than in the method described in the invention.

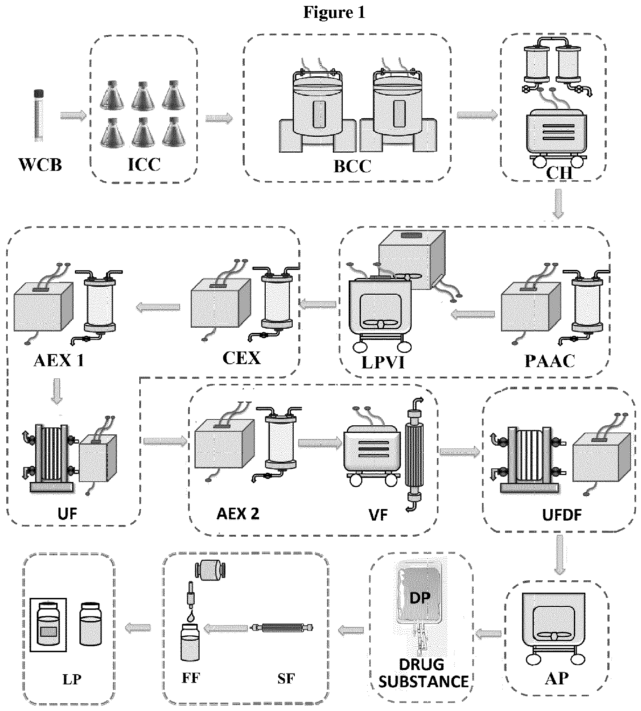

In a first aspect, the present invention provides a pharmaceutical composition comprising an antibody comprising in its light chain a LCDR1 with the amino acid sequence of SEQ ID NO: 1, a LCDR2 with the amino acid sequence of SEQ ID NO: 2, and a LCDR3 with the amino acid sequence of SEQ ID NO: 3 and comprising in its heavy chain a HCDR1 with the amino acid sequence of SEQ ID NO: 4, a HCDR2 with the amino acid sequence of SEQ ID NO: 5, and a HCDR3 with the amino acid sequence of SEQ ID NO: 6, wherein less than 0.7 wt%, preferably less than 0.6 wt%, more preferably less than 0.5 wt% of the antibody in the composition is aggregated, and/or wherein the antibody is obtainable by a process comprising (Figure 1):

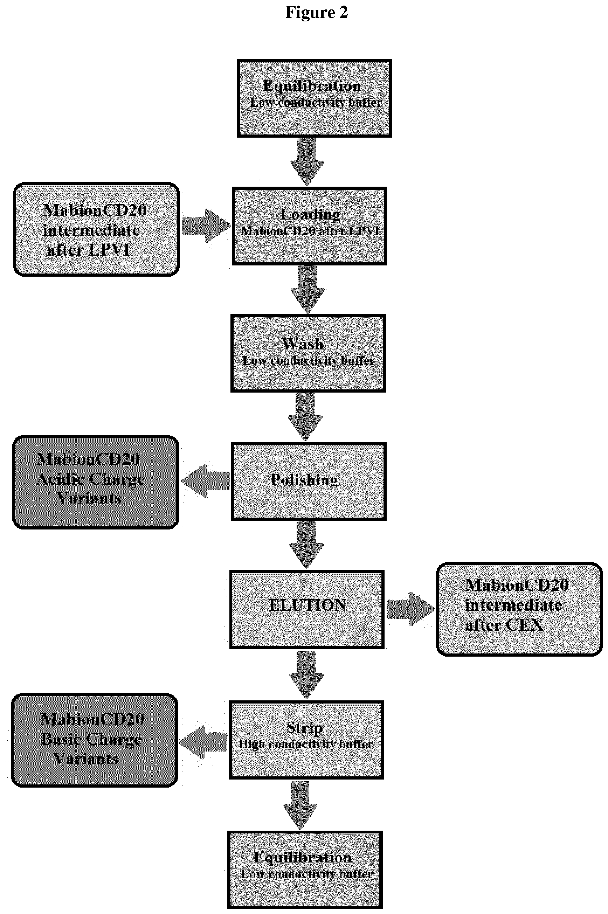

> (i) incubating a mammalian cell comprising a nucleic acid encoding the antibody in a culture medium in suspension culture, wherein the mammalian cell secretes the antibody into the culture medium;
> (ii) separating the antibody from the culture medium;
> (iii) solubilizing the antibody in a buffer; and
> (iv) purifying the antibody by binding to a cation-exchange chromatography resin and eluting a fraction of the antibody from the resin, comprising less than 10% of basic charge variants of the antibody; the flow chart presenting the method of purification is presented on Figure 2

wherein
the pH of the buffer used in solubilizing and purifying the antibody is between 5 and 8, the conductivity is between 1 and 50 mS/cm.

The antibody defined by its CDR sequences in the first aspect of the invention refers to the anti-CD20 antibody rituximab. The terms Mabion CD20, Mabion CD20DP and Mabion CD20 DS refer to antibody preparations of the present invention comprising an antibody with an identical sequence to rituximab.

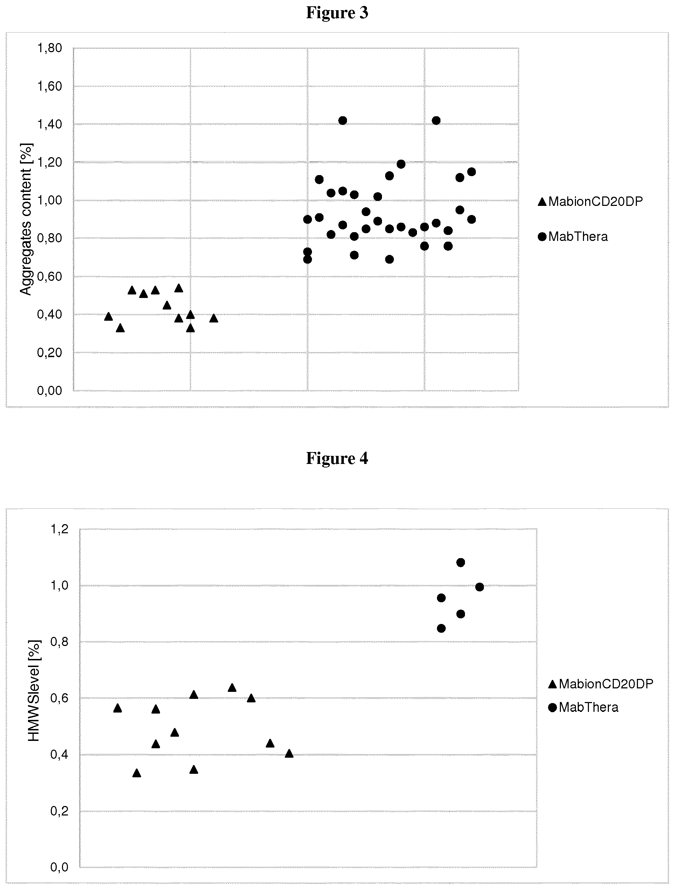

The level of antibody aggregation can be measured by art known methods, including SEC-HPLC (see Figure 2) and SEC-MALLS (see Figure 3 and Figure 4). The preferred method is SEC-HPLC.

Preferably, the mammalian cells used in step (i) are hybridomas which includes a B cell fused to an immortalized cell.

In a preferred embodiment of the first aspect of the invention, the antibody is obtainable by a process comprising:

> (iv) purifying the antibody by binding to a cation-exchange chromatography resin and eluting a fraction of the antibody from the resin, comprising less than 10% of basic charge variants of the antibody; the flow chart presenting the method of purification is presented on Figure 2

wherein the conductivity is between 1 and 50 mS/cm.

In a preferred embodiment of the first aspect of the present invention, the incubation step (i) is performed in a cell culture bag.

In a preferred embodiment of the first aspect of the present invention, the incubation step comprises orbital shaking mixing.

In a preferred embodiment of the first aspect of the present invention, the incubation step does not comprise stirring with a stirrer.

In a preferred embodiment of the first aspect of the present invention, the purification step comprises or consists of the following steps:

(a) affinity chromatography, preferably Protein A affinity purification;
(b) virus inactivation at pH above 3.1, when the exposure time is longer than 30 minutes;
(c) cation exchange chromatography, when affinity chromatography product (step(a)) does not contain 100% of antibody subjected for cation exchange chromatography;
(d) anion exchange chromatography;
(e) ultrafiltration; and/or
(f) virus filtration,

wherein one or more of the above steps can be repeated one or more times.

Without wishing to be bound by theory the inventors believe that affinity chromatography, in particular Protein A affinity chromatography, contributes to the aggregate removal. The main step of removal aggregates is cation exchange chromatography, which apart from the removal of almost all aggregates, changes the composition of isoforms, which makes it possible to establish a balance between aggregates and monomer on very low level.

In a preferred embodiment, the purification step comprises or consists of affinity chromatography, preferably Protein A affinity purification, and/or cation exchange chromatography.

In a more preferred embodiment, the purification step comprises or consist of cation exchange chromatography.

In a preferred embodiment of the first aspect of the present invention, the purification step, in particular cation exchange chromatography is performed by a strong cation exchanger, preferably, wherein the strong cation exchanger comprises a $SO_3^-$ functional group. The term "strong cation exchanger" is used in its meaning known in the art and refers to cation exchangers that maintain the charge on their matrix over a wide range of pH values and can therefore be used with a wide range of buffers.

In a preferred embodiment the virus inactivation is performed at a pH of about 3.2.

In a preferred embodiment the virus inactivation is performed for a duration of 30 min to 2h, preferably 30 min to 1h, more preferably for about 1h.

In a preferred embodiment steps (c) to (f) are performed at a pH of 6.5.

In a preferred embodiment of the first aspect of the present invention, less than 0.6 wt%, preferably less than 0.5 wt% of the antibody in the composition is aggregated. In a more preferred embodiment less than 0.45, 0.4, 0.35, 0.3 or 0.25 wt% of the antibody in the composition is aggregated. The preferred method to determine the level of aggregation of the antibody is SEC-HPLC. An example of such a measurement can be seen in figure 3.

In a preferred embodiment of the first aspect of the invention the conductivity is between 1 and 50, 1 and 40, 1 and 20, 1 and 15 and preferably 1 to 7 mS/cm.

In a preferred embodiment of the first aspect of the invention at least one of steps (i) to (iv), i.e. (i), (ii), (iii) and (iv), comprise the use of single-use disposables. In a preferred embodiment of the first aspect of the invention at least one of steps (a) to (f), i.e. (a), (b), (c), (d), (e) and (f), comprise the use of single-use disposables. Non-limiting examples of single-use disposables are single-use cell culture or storage bags, disposable pump head and single-use membranes.

In a preferred embodiment of the first aspect of the present invention the antibody is not contacted with stainless steel during steps (i) to (iv). Without wishing to be bound by theory, the inventors believe that contact with stainless steel surfaces increases the aggregation levels of antibodies. It is therefore preferred to avoid contact of the antibodies with stainless steel surfaces to avoid an increase of antibody aggregation levels.

In a preferred embodiment of the first aspect of the present invention, the pharmaceutical composition is further comprising at least one pharmaceutically acceptable carrier.

In a preferred embodiment of the first aspect of the present invention, the pharmaceutical composition is further comprising at least one further active agent. Preferably the further active agent is selected from the group comprising teriflunomide, fingolimod and .dimethyl fumarate

In a second aspect, the present invention provides the pharmaceutical composition according to the first aspect of the invention for use in the treatment of multiple sclerosis.

In a preferred embodiment of the second aspect of the present invention, multiple sclerosis is selected from the group consisting of clinically isolated syndrome (CIS), relapsing-remitting multiple sclerosis (RRMS), primary progressive multiple sclerosis (PPMS), progressive-relapsing multiple sclerosis (PRMS) and secondary progressive multiple sclerosis (SPMS).

**Examples**

**Example 1: Production and purification process of Mabion CD20DP**

In figure 1 a process flow chart depicts the different steps in the production and purification of Mabion CD20DP. The different steps are explained in the following.

Cultivation of hybridoma cells

The working cell bank (WCB) of hybridoma cells is used for a starter cell culture (ICC) that is used to start the industrial scale cell culture in a bioreactor (BCC).

The bioreactor cell culture is conducted in bioreactors in fed-batch mode with orbital shaking as a way of mixing, in single-use bags. Oxygenation of the cell culture, pH, temperature, shaking frequency, gas flow, gas composition and pressure in the bioreactor bag are monitored. The oxygenation is regulated by shaking frequency, gas flow and oxygen concentration in the gas mixture. The minimum oxygenation of the bioreactor cell culture is 20% and depends on the day of culture. pH of the culture is in the range 6.5 to 7.5 during the whole culture. The bioreactor cell culture starts with the aseptic transfer of sterile serum-free chemically-defined medium to the bioreactor bag. The medium is warmed up to 36-38°C and shaken to increase oxygenation. When the medium temperature is 36-38°C, the bag containing the inoculum is aseptically connected to the bioreactor bag, and the inoculum is transferred to the warmed medium. The bioreactor cell culture is sampled for cell viability and density calculation. Also, concentration of MabionCD20DS is determined on taken samples. During the cell culture, medium and supplements are added to the bioreactor based on the cell density. Therefore, the volume increases during the cultivation. When culture density exceeds $0.8 \times 10^6$ cells per mL, but not earlier than on the seventh day, half of the cell culture is transferred to the second bioreactor bag containing warmed and sterile serum-free chemically-defined medium. When the transfer ends, the medium is added to the donor bioreactor to complement the existing volume to the volume which was before the transfer. Since the bioreactor cell culture split, two bioreactors are controlled, sampled and supplemented with new portions of medium and supplements scheme. Bioreactor cell culture is completed, and cell harvest starts when average cell viability decreases below 40% but no less than 30%. After cell harvest (CH) the resulting mixture is further purified by depth filtration to separate the cells from the culture fluid.

Protein A affinity chromatography (PAAC)

The step objective is to reduce process associated impurities (HCP, HCDNA, Aggregates).

Protein A affinity chromatography is conducted in isocratic mode. The chromatography column used is packed with highly cross-linked agarose medium with alkali-resistant protein A. Every chromatography run consists of binding MabionCD20 to the resin, washing off the process related impurities and eluting MabionCD20 with low pH buffer (sodium citrate buffer; pH 3.5). All steps are performed at a constant flow velocity. The intermediate product after protein A affinity chromatography (eluate) is collected in a single-use bag. The eluate is stored at 2-8°C before pooling and the next process step. The protein A affinity chromatography step consists of multiple runs, depending on the amount of product for purification. The maximum and minimum load of protein on the column is the regulation factor.

Low pH Virus inactivation (LPVI)

The step objective is to inactivate potential virus contaminants.

The low pH virus inactivation step starts with pooling the intermediate product after protein A affinity chromatography in a single-use bag with the impeller and single-use pH probe. To achieve inactivation of the viruses in the sample, the pH is lowered to the target value (pH 3.2) with acid. The product is mixed until pH reaches stable target value and it is transferred to a new single-use bag with impeller and pH probe. During the transfer, MabionCD20DS is filtrated through a single-use filter (0.2 $\mu$m filter) to remove HCP precipitate caused by low pH. When the whole material is in the new bag the incubation time starts. After a set time (less than 90 minutes, preferably 60 to 70 minutes), incubation ends with the addition of neutralization buffer (pH 7.5 to 9). When the target pH of about pH 6.5 is stable, MabionCD20DS is diluted with water for injection (WFI), highly purified or purified water. Then MabionCD20DS is filtered in portions through a 0.2 $\mu$m grade filter to the sterile single-use bags. Bags containing intermediate after low pH virus inactivation are stored at 2-8 °C.

Cation exchange chromatography (CEX)

The step objective is to control the proportion of charge variants in MabionCD20DS charge profile to obtain values set in the specification of MabionCD20DP. The CEX process is aimed firstly at reducing the amount of acidic variants and secondly to increase conductivity of the elution buffer which leads to elution of the product with decreased content of basic charge variants.

The cation exchange chromatography is conducted in bind and elute mode. The chromatography column is packed with the methacrylate cation exchanger medium containing sulfonate functional groups. Every chromatography run consists of binding intermediate product after low pH virus inactivation to the resin, washing the column with phosphate buffer, conductivity-based polishing fraction which reduces acidic charge variants followed by conductivity-based elution of intermediate product after cation exchange chromatography. The washing buffer used should buffer in the range of 5.5 and 8 pH. Suitable buffers for washing the column include sodium phosphate buffer and sodium phosphate buffer with NaCl, which is used for gradients. Basic charge variants are removed in conductivity-based strip fraction with phosphate buffer with sodium chloride. This buffer should also buffer in the range of 5.5 and 8 pH. Resin is then equilibrated with phosphate buffer. Purification of a single batch is performed in multiple cycles, depending on the amount of product for purification. The maximum and minimum load of protein on the column is the regulation factor.

Anion Exchange Chromatography 1 (AEX1)

The step objective is to reduce process associated impurities (HCP, HCDNA, Aggregates).

The chromatography is carried out in flow-through mode where MabionCD20DS does not bind and flows through the resin during loading, while process-related impurities bind to the resin. The step is performed on Sartobind Q disposable membrane adsorber (Sartorius) containing stabilized resin on reinforced cellulose with a strong anion ligand (quaternary ammonium).

Before the purification, the intermediate product after cation exchange chromatography is diluted in a single-use bag having an impeller, with phosphate buffer (sodium phosphate buffer) to lower the conductivity of intermediate product after cation exchange chromatography below 7 mS/cm. The disposable chromatography set with disposable anion exchange capsule is rinsed with phosphate buffer to prepare the membrane (reinforced cellulose with a strong anion ligang (quarternary ammonium) and flush leachable compounds.

The material is passed through the membrane via a disposable tube placed in a peristaltic pump. After the whole material is processed, the membrane is rinsed with phosphate buffer to recover intermediate product after anion exchange chromatography from the void volume of membrane and tubes. Intermediate product after anion exchange chromatography is collected in a sterile disposable bag, after which it is directly taken to ultrafiltration (next step).

Ultrafiltration

The step objective is to concentrate the intermediate MabionCD20DS.

It is conducted using a single-use ultrafiltration cassette (Sartorius 30kDa Sartocon Slice). Flow in the system is generated by a membrane pump with a disposable pump head.

Prior to product loading, the system is rinsed with phosphate buffer to prepare the cassette and flush leachable compounds. Intermediate product after anion exchange chromatography 1 step is constantly transferred to the retentate single-use bag with a flow rate that allows maintaining a constant volume of material on the ultrafiltration system. The ultrafiltration is conducted at 1,0 bar (+/- 0,2) transmembrane pressure.

Anion Exchange Chromatography 2 (AEX 2)

The step objective is to remove potential presence virus contaminants.

The chromatography is carried out in flow-through mode where MabionCD20 does not bind and flows through the resin during loading while viruses (if present) bind to the resin. The step is performed on disposable membrane adsorber-stabilized resin on reinforced cellulose with strong anion ligand (quaternary ammonium).

Before the purification, the intermediate product after ultrafiltration is diluted in a single-use bag with an impeller, with phosphate buffer to set the target protein concentration and conductivity (below 50 mS/cm, preferably below 12 mS/cm).

The diluted intermediate product after ultrafiltration is passed through the membrane via a disposable tube installed on a peristaltic pump. When the whole intermediate is processed, the membrane is rinsed with phosphate buffer to recover intermediate product after anion exchange chromatography 2 from the void volume of the membrane, prefilter and tubes. Intermediate product after AEX2 is collected into a sterile disposable bag, which is directly taken for virus filtration.

Virus filtration (VF)

The step objective is to remove potential presence virus contaminants.

The pre-filter and virus removal filter are rinsed with phosphate buffer to prepare the filters and remove leachable compounds. The membrane pump with disposable pump-head generates the flow required for the virus filtration. The intermediate product after anion exchange chromatography 2 is filtered under a constant pressure to a new disposable bag with the impeller. After filtration, filters are rinsed with phosphate buffer to recover intermediate product after virus filtration from the void volume of pre-filter and virus removal filter. The bags with intermediate product after virus filtration are stored at 2 8°C.

Ultrafiltration with Diafiltration (UFDF)

The step objective is to concentrate the intermediate MabionCD20 and buffer exchange.

The process step consists of the ultrafiltration and diafiltration which are conducted subsequently using single-use ultrafiltration cassette. In UFDF the intermediate product after VF is concentrated. When the designated concentration is reached the diafiltration starts and the buffer is exchanged from phosphate buffer to diafiltration buffer (such as NaCl, trisodium citrate dehydrate).

Prior to loading the intermediate product after virus filtration, the system is rinsed with diafiltration buffer to prepare the cassette and flush leachable compounds. The pressure is controlled by the pump speed and valve on the retentate line. The UFDF step is conducted at set TMP (transmembrane pressure).

Intermediate product after virus filtration step is continuously transferred to the retentate disposable bag at a flow rate that allows to maintain a constant volume of product on the ultrafiltration system. When the entire intermediate product after VF is concentrated the single-use bag with diafiltration buffer is connected to the system. The diafiltration is conducted at constant volume of the retentate without changing the concentration of MabionCD20. To finish the diafiltration part of the UFDF, set buffer exchanges are required. The intermediate product after ultrafiltration/diafiltration is collected from the system to the single-use bag through the sterilization grade filter. The ultrafiltration/diafiltration system is flushed with diafiltration buffer to recover remaining intermediate product after UFDF. The buffer from flushing is collected through the same sterilization grade filter. The bag is stored at 2-8°C.

Adding polysorbate and dilution (AP)

The step objective is to add formulation agent.

The single-use bag containing intermediate product after ultrafiltration/diafiltration step is aseptically connected to the single-use bag with polysorbate 80 (sterile solution) dissolved in the diafiltration buffer. The amount of polysorbate 80 and diafiltration buffer used for the preparation of the polysorbate 80 solution is calculated based on the concentration of MabionCD20 and its mass, based on the formula . $M_p = C_p \times V \times 0.07$, with Mp = mass of polysorbate and V = volume of solution

The solution of polysorbate 80 in the diafiltration buffer is added to the intermediate product after UFDF to achieve target concentration of polysorbate 80 of 9.7 to 10.5 mg/ml per 1 mL of MabionCD20DS and to dilute intermediate product after UFDF to target concentration. Amounts of intermediate product after UFDF and polysorbate 80 solutions are verified before and after addition step. MabionCD20DS is storage in 2-8 °C.

**Example 2: Determination of Mabion CD20DP aggregate levels**

Size Exclusion Chromatography-HPLC (SEC-HPLC)

The analysis is performed with use of 290 Å, 5 μm size exclusion column on an isocratic gradient according to Table 1:

*Table 1: Parameters of SEC-HPLC analysis*

| Parameter | Value |
|---|---|
| Flow rate | 0.4 mL/min (Buffer A 100%) |
| Column thermostating temperature | 25°C |
| UV detection | 280 nm |
| Time | 40 min |

(continued)

| Parameter | Value |
|---|---|
| Pressure limit | 85 bar |
| Buffer A composition is presented in Table 2: | |

*Table 2: Composition of SEC-HPLC mobile phases*

| REAGENT | CONCENTRATION [M] IN WATER | pH |
|---|---|---|
| Sodium chloride | 0.300 | 6.7 |
| Disodium hydrogen phosphate | 0.012 | |
| Disodium hydrogen phosphate | 0.038 | |
| Sodium azide | 0.008 | |

The area of aggregate and main peak is measured based on the automatic integration of the peaks. Percent (%) content of aggregate and monomer for individual injection is calculated based on Formula 1.

**Formula 1: Calculation of the % aggregate/monomer content**

$$\%\text{aggregate/monomer} = (\text{area aggregate/monomer area}) \,/\, (\text{aggregate area} + \text{main peak area})$$

The mean % of aggregate and monomer for a sample is calculated based on Formula 2.

**Formula 2: Calculation of the aggregate and monomer content and the retention time**

$$x_{\text{śr}} = (x_1 + x2)/2$$

where:

$x_1$ - is the content/retention time for peak during the 1st injection

$x_2$ - is the content/retention time for peak during the 2nd injection

$x'_{\text{śr}}$ - is the mean content/retention time for peak

Size Exclusion Chromatography-Multi-Angle Laser Light Scattering (SEC-MALLS)

Size Exclusion Chromatography-Multi-Angle Laser Light Scattering (SEC-MALLS) is an established method for assessment of protein quality and characterization. SEC-MALLS is a useful methodology to separate and characterize protein aggregates in native solution conditions. MALLS is especially relevant for high molecular weight species.
Analysis of protein aggregation is carried out by Size Exclusion Chromatography with UV detection. Molecular weight analysis of peaks separated by Size Exclusion Chromatography is carried out by Multi-Angle-Laser-Light-Scattering (SEC-MALLS).
The separation of the samples is performed with size exclusion chromatography with refractive index (RI), UV (280 nm) and MALLS (637 nm) detection. The separator column TSKgel (5 $\mu$m 7.8 x 300 mm) is used. The eluent is 100 mM sodium phosphate buffer with 250 mM sodium chloride, pH 6.8. The samples are applied by injecting 20 $\mu$L at 2.5 mg/mL on the column in duplicates. The samples are diluted in the diluent. The samples are separated using an isocratic elution at 25°C (column temperature) and 35°C (RI detector temperature) with a flow rate of 0.5 mL/min.

The area percentage of monomer and multimer are determined after UV detection at 280 nm. The mass of each peak is determined from the MALLS signal. The protein concentration is determined using the RI signal and the dn/dc value of 0.186.

he elugram overlays of the replicate and the diluent measurements of the absorption at 280 nm are checked. The area percentage (area%) and the deviation between duplicates of monomer, multimers (HMW) and lower molecular weight (LMW) species according to UV detection at 280 nm are determined. An overlay of calculated MW and RI signal versus the elution volume is presented.

Claims

1. Pharmaceutical composition comprising an antibody comprising in its light chain a LCDR1 with the amino acid sequence of SEQ ID NO: 1, a LCDR2 with the amino acid sequence of SEQ ID NO: 2, and a LCDR3 with the amino acid sequence of SEQ ID NO: 3 and comprising in its heavy chain a HCDR1 with the amino acid sequence of SEQ ID NO: 4, a HCDR2 with the amino acid sequence of SEQ ID NO: 5, and a HCDR3 with the amino acid sequence of SEQ ID NO: 6, wherein less than 0.7 wt% of the antibody in the composition is aggregated, and/or wherein the antibody is obtainable by a process comprising:

   (i) incubating a mammalian cell comprising a nucleic acid encoding the antibody in a culture medium in suspension culture, wherein the mammalian cell secretes the antibody into the culture medium;
   (ii) separating the antibody from the culture medium;
   (iii) solubilizing the antibody in a buffer; and
   (iv) purifying the antibody by binding to a cation-exchange chromatography resin and eluting a fraction of the antibody from the resin, comprising less than 10% of basic charge variants of the antibody;

   wherein
   the pH of the buffer used in solubilizing and purifying the antibody is between 5 and 8, the conductivity is between 1 and 50 mS/cm.

2. The pharmaceutical composition according to any of the preceding claims, wherein the purification step comprises or consists of the following steps:

   (a) affinity chromatography, preferably Protein A affinity purification;
   (b) virus inactivation at pH above 3.1;
   (c) cation exchange chromatography;
   (d) anion exchange chromatography;
   (e) ultrafiltration; and/or
   (f) virus filtration,

   wherein one or more of the above steps can be repeated one or more times.

3. The pharmaceutical composition according to claims 1 or 2, wherein at least one of steps (i) to (iv) of claim 1 and/or (a) to (f) of claim 2 comprises the use of single-use disposables.

4. The pharmaceutical composition according to claim 1, wherein the antibody is not contacted with stainless steel during steps (i) to (iv).

5. The pharmaceutical composition according to any of the preceding claims, wherein the cation exchange chromatography step is performed by a strong cation exchanger, preferably, wherein the strong cation exchanger comprises a $SO_3^-$ functional group.

6. The pharmaceutical composition according to any of the preceding claims, wherein less than 0.6 wt%, preferably less than 0.5 wt% of the antibody in the composition is aggregated.

7. The pharmaceutical composition according to any of the preceding claims, further comprising at least one pharmaceutically acceptable carrier.

8. The pharmaceutical composition according to any of the preceding claims, further comprising at least one further active agent.

9. Pharmaceutical composition according to any of the preceding claims for use in the treatment of multiple sclerosis.

10. The pharmaceutical composition for use according to claim 9, wherein multiple sclerosis is selected from the group consisting of clinically isolated syndrome (CIS), relapsing-remitting multiple sclerosis (RRMS), primary progressive multiple sclerosis (PPMS), progressive-relapsing multiple sclerosis (PRMS) and secondary progressive multiple sclerosis (SPMS).

# Figure 1

## Figure 2

**Figure 3**

**Figure 4**

## Figure 5

## Figure 6

Europäisches
Patentamt

European
Patent Office

Office européen
des brevets

# EUROPEAN SEARCH REPORT

Application Number

EP 18 46 1619

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KYOUNG HOON LEE ET AL: "Analytical similarity assessment of rituximab biosimilar CT-P10 to reference medicinal product", MABS, vol. 10, no. 3, 6 March 2018 (2018-03-06), pages 380-396, XP055577660, US ISSN: 1942-0862, DOI: 10.1080/19420862.2018.1433976 * table 2 * | 1-10 | INV. A61K39/395 C07K16/06 C07K16/28 |
| X | WO 2014/207763 A1 (CADILA HEALTHCARE LTD [IN]) 31 December 2014 (2014-12-31) * figure 8; examples 1,2 * | 1-10 | |
| X | R. T. NAISMITH ET AL: "Rituximab add-on therapy for breakthrough relapsing multiple sclerosis: A 52-week phase II trial", NEUROLOGY, vol. 74, no. 23, 8 June 2010 (2010-06-08), pages 1860-1867, XP055004375, ISSN: 0028-3878, DOI: 10.1212/WNL.0b013e3181e24373 * figures 1-3 * | 9,10 | TECHNICAL FIELDS SEARCHED (IPC) A61K C07K |
| X | WO 2009/058812 A1 (GENENTECH INC [US]; LEBRETON BENEDICTE ANDREE [US] ET AL.) 7 May 2009 (2009-05-07) * figure 1; example 1; tables 1,2 * | 1-10 | |
| A | WO 2014/161940 A1 (MABXIENCE S A [UY]; MABXIENCE SWITZERLAND [CH]) 9 October 2014 (2014-10-09) * the whole document * | 1-10 | |

-/--

The present search report has been drawn up for all claims

| Place of search | Date of completion of the search | Examiner |
|---|---|---|
| Munich | 5 April 2019 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 18 46 1619

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/034726 A1 (LAB FRANCAIS DU FRACTIONNEMENT [FR]) 10 March 2016 (2016-03-10) * figure 1 * | 1-10 | |
| A | WO 2012/160530 A1 (REDDYS LAB LTD DR [IN]; JAHAGIRDAR KISHORE [IN]; GUPTA NEERU [IN]) 29 November 2012 (2012-11-29) * examples 1-3 * | 1-10 | |

TECHNICAL FIELDS
SEARCHED      (IPC)

The present search report has been drawn up for all claims

| Place of search | Date of completion of the search | Examiner |
|---|---|---|
| Munich | 5 April 2019 | Cilensek, Zoran |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 46 1619

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2019

| Patent document cited in search report | Publication date | Patent family member(s) | | Publication date |
|---|---|---|---|---|
| WO 2014207763 A1 | 31-12-2014 | AR | 096713 A1 | 27-01-2016 |
| | | AU | 2014300486 A1 | 26-11-2015 |
| | | BR | 112015031196 A2 | 25-07-2017 |
| | | CA | 2911874 A1 | 31-12-2014 |
| | | CN | 105263947 A | 20-01-2016 |
| | | EA | 201592192 A1 | 29-07-2016 |
| | | EP | 3013849 A1 | 04-05-2016 |
| | | HK | 1217344 A1 | 06-01-2017 |
| | | IL | 242649 A | 31-03-2016 |
| | | JP | 6039828 B2 | 07-12-2016 |
| | | JP | 2016525500 A | 25-08-2016 |
| | | KR | 20160003311 A | 08-01-2016 |
| | | MX | 350610 B | 11-09-2017 |
| | | NZ | 714079 A | 26-05-2017 |
| | | SG | 11201509502X A | 30-12-2015 |
| | | TW | 201512217 A | 01-04-2015 |
| | | US | 2016115195 A1 | 28-04-2016 |
| | | WO | 2014207763 A1 | 31-12-2014 |
| | | ZA | 201508258 B | 29-03-2017 |
| WO 2009058812 A1 | 07-05-2009 | AR | 069097 A1 | 30-12-2009 |
| | | AU | 2008318865 A1 | 07-05-2009 |
| | | BR | PI0817182 A2 | 17-03-2015 |
| | | CA | 2703279 A1 | 07-05-2009 |
| | | CN | 101889025 A | 17-11-2010 |
| | | CN | 103554215 A | 05-02-2014 |
| | | CN | 105315323 A | 10-02-2016 |
| | | CO | 6280422 A2 | 20-05-2011 |
| | | CY | 1116129 T1 | 08-02-2017 |
| | | DK | 2215117 T3 | 02-03-2015 |
| | | DK | 2565206 T3 | 06-06-2016 |
| | | DK | 2840090 T3 | 23-04-2018 |
| | | EP | 2215117 A1 | 11-08-2010 |
| | | EP | 2565206 A2 | 06-03-2013 |
| | | EP | 2840090 A1 | 25-02-2015 |
| | | EP | 3441402 A1 | 13-02-2019 |
| | | ES | 2533266 T3 | 08-04-2015 |
| | | ES | 2572958 T3 | 03-06-2016 |
| | | ES | 2666170 T3 | 03-05-2018 |
| | | HK | 1143821 A1 | 31-07-2015 |
| | | HK | 1182401 A1 | 23-06-2017 |
| | | HK | 1206753 A1 | 15-01-2016 |
| | | HK | 1221234 A1 | 26-05-2017 |
| | | HR | P20150282 T1 | 19-06-2015 |
| | | HR | P20180943 T1 | 10-08-2018 |
| | | HU | E024877 T2 | 29-02-2016 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 18 46 1619

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2019

| Patent document cited in search report | Publication date | Patent family member(s) | | Publication date |
|---|---|---|---|---|
| | | HU | E027668 T2 | 28-11-2016 |
| | | HU | E037409 T2 | 28-08-2018 |
| | | JP | 5237382 B2 | 17-07-2013 |
| | | JP | 5885864 B2 | 16-03-2016 |
| | | JP | 2011502161 A | 20-01-2011 |
| | | JP | 2013173747 A | 05-09-2013 |
| | | JP | 2015145371 A | 13-08-2015 |
| | | JP | 2015155406 A | 27-08-2015 |
| | | JP | 2017019790 A | 26-01-2017 |
| | | KR | 20100086015 A | 29-07-2010 |
| | | KR | 20140015166 A | 06-02-2014 |
| | | KR | 20150008503 A | 22-01-2015 |
| | | ME | 02101 B | 20-10-2015 |
| | | NZ | 584839 A | 27-07-2012 |
| | | PE | 14342009 A1 | 17-10-2009 |
| | | PH | 12013501128 A1 | 02-12-2015 |
| | | PL | 2215117 T3 | 30-06-2015 |
| | | PL | 2565206 T3 | 31-08-2017 |
| | | PL | 2840090 T3 | 31-07-2018 |
| | | PT | 2215117 E | 01-04-2015 |
| | | RU | 2010121816 A | 10-12-2011 |
| | | SG | 175597 A1 | 28-11-2011 |
| | | SG | 10201401690X A | 27-06-2014 |
| | | SI | 2215117 T1 | 31-03-2015 |
| | | SI | 2840090 T1 | 31-05-2018 |
| | | TW | 200927289 A | 01-07-2009 |
| | | TW | 201512216 A | 01-04-2015 |
| | | US | 2009148435 A1 | 11-06-2009 |
| | | US | 2015056196 A1 | 26-02-2015 |
| | | US | 2018118781 A1 | 03-05-2018 |
| | | WO | 2009058812 A1 | 07-05-2009 |
| | | ZA | 201002850 B | 27-07-2011 |
| | | ZA | 201102169 B | 27-12-2012 |
| WO 2014161940 A1 | 09-10-2014 | AR | 095977 A1 | 25-11-2015 |
| | | AU | 2014247034 A1 | 01-10-2015 |
| | | BR | 112015025106 A2 | 10-10-2017 |
| | | CA | 2907766 A1 | 09-10-2014 |
| | | CN | 105121469 A | 02-12-2015 |
| | | EP | 2981553 A1 | 10-02-2016 |
| | | HK | 1221472 A1 | 02-06-2017 |
| | | JP | 2016523812 A | 12-08-2016 |
| | | KR | 20150138273 A | 09-12-2015 |
| | | RU | 2015147187 A | 23-05-2017 |
| | | SG | 10201708088W A | 30-10-2017 |
| | | SG | 11201507815X A | 29-10-2015 |

EPO FORM P0459

For more details about this annex : see Official Journal of the European Patent Office, No. 12/82

page 2 of 3

**ANNEX TO THE EUROPEAN SEARCH REPORT
ON EUROPEAN PATENT APPLICATION NO.**                    EP 18 46 1619

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2019

| Patent document cited in search report | Publication date | Patent family member(s) | | Publication date |
|---|---|---|---|---|
| | | TW | 201444863 A | 01-12-2014 |
| | | US | 2016060349 A1 | 03-03-2016 |
| | | UY | 35517 A | 31-10-2014 |
| | | WO | 2014161940 A1 | 09-10-2014 |
| WO 2016034726 A1 | 10-03-2016 | CA | 2959947 A1 | 10-03-2016 |
| | | EP | 3189075 A1 | 12-07-2017 |
| | | FR | 3025515 A1 | 11-03-2016 |
| | | US | 2017274299 A1 | 28-09-2017 |
| | | WO | 2016034726 A1 | 10-03-2016 |
| WO 2012160530 A1 | 29-11-2012 | EP | 2714713 A1 | 09-04-2014 |
| | | US | 2014107321 A1 | 17-04-2014 |
| | | WO | 2012160530 A1 | 29-11-2012 |

EPO FORM P0459

For more details about this annex : see Official Journal of the European Patent Office, No. 12/82

page 3 of 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030118592 A
- US 20030133939 A
- US 5939598 A, Kucherlapati & Jakobovits

**Non-patent literature cited in the description**

- **NAISMITH R.T. et al.** *Neurology,* 2010, vol. 74, 1860-1867
- **CROSS et al.** *TherAdvNeurolDisord,* 2012, vol. 5 (6), 311-319
- **CASTILLO-TRIVINO et al.** *PLOS One,* 2013, vol. 8 (7), e66308
- **RUNKEL et al.** *PharmRes,* 1998, vol. 15 (4), 641-9
- **ROSENBERG.** *AAPS J.,* 2006, vol. 8 (3), E501-07
- A multilingual glossary of biotechnological terms: (IUPAC Recommendations). Helvetica Chimica Acta, 1995
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **KONTERMANN.** *MAbs,* 2014, vol. 4, 182-197
- **DEISENHOFER.** *Biochemistry,* 1981, vol. 20, 2361-2370
- **DESMYTER et al.** *Nat. Structure Biol.,* 1996, vol. 3, 803-811
- **KUFER et al.** *Trends Biotechnol.,* 2004, vol. 22, 238-244
- **BINZ H.K. et al.** Engineering novel binding proteins from nonimmunoglobulin domains. *Nat. Biotechnol.,* 2005, vol. 23 (10), 1257-1268
- **HOLLIGER P. et al.** *Proc. Natl. Acad. Sci. U.S.A.,* 1993, vol. 90 (14), 6444-6448
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies, A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82